# EUROPÄISCHE PATENTSCHRIFT

(11) **EP 1 203 094 B1**
(45) Veröffentlichungstag und Bekanntmachung des Hinweises auf die Patenterteilung: **01.09.2004**
(21) Anmeldenummer: 00940212.4
(22) Anmeldetag: 22.05.2000
(51) Int. Cl.: C12Q 1/68

(54) **VERFAHREN ZUR MARKIERUNG VON FESTEN, FLÜSSIGEN UND GASFÖRMIGEN SUBSTANZEN**
METHOD FOR MARKING SOLID, LIQUID AND GASEOUS SUBSTANCES
PROCEDE POUR LE MARQUAGE DE SUBSTANCES SOLIDES, LIQUIDES ET GAZEUSES

(30) Priorität: 22.07.1999 DE 19934573
(43) Veröffentlichungstag der Anmeldung: 08.05.2002
(73) Patentinhaber: November Aktiengesellschaft Gesellschaft für Molekulare Medizin, 91056 Erlangen (DE)
(72) Erfinder: BERTLING, Wolf, D-91056 Erlangen (DE); KOSAK, Hans, D-53123 Bonn (DE)
(74) Vertreter: Gassner, Wolfgang, Dr.
(86) Internationale Anmeldenummer: PCT/DE2000/001674
(87) Internationale Veröffentlichungsnummer: WO 2001/007645

(56) Entgegenhaltungen:
- EP-A- 0 794 261
- WO-A-90/14441
- WO-A-94/04918
- WO-A-94/12632
- WO-A-97/07235
- WO-A-99/13102
- US-A- 5 866 336
- SANDHU ET AL: "Dual assymetric PCR: One-step construction of synthetic genes" BIOTECHNIQUES,US,EATON PUBLISHING, NATICK, Bd. 12, Nr. 1, 1992, Seiten 14-16, XP002134139 ISSN: 0736-6205
- SANO T ET AL: "Deoxyribonucleic acids as unique markers in molecular detection" GENETIC ANALYSIS: BIOMOLECULAR ENGINEERING,US,ELSEVIER SCIENCE PUBLISHING, Bd. 14, Nr. 2, 1. Juli 1997 (1997-07-01), Seiten 37-40, XP004126263 ISSN: 1050-3862

## Beschreibung

Die Erfindung betrifft ein Verfahren zur Markierung von festen, flüssigen und gasförmigen Substanzen.

Nach dem Stand der Technik sind verschiedene Verfahren bekannt, bei denen zur Markierung Nukleinsäuresequenzen benutzt werden.

Bei dem aus der WO 90/14441 bekannten Verfahren wird eine vorgegebene spezifische Nukleinsäuresequenz amplifiziert und anschließend identifiziert.- Eine komplexe Markierung nach Art eines Codes ist damit nicht realisierbar.

In der WO 91/17265 ist ein Verfahren zur Markierung eines Materials mit Mikrospuren von DNA beschrieben. Die in geringsten Mengen vorhandene DNA wird durch eine PCR amplifiziert. Die Identifizierung der DNA erfolgt durch Sequenzierung. Der Nachweis der zur Markierung eingesetzten DNA ist langwierig und aufwendig.

Bei den in der US 5,866,336 offenbarten Verfahren wird durch Energie-Transfer zwischen Donor- und Akzeptor-Molekülen eine Ausbildung intra- oder intermolekularer Hybride nachgewiesen.

Bei der WO 94/04918 wird zur Markierung einer Flüssigkeit eine an einen Partikel gebundene Nukleinsäure benutzt. Die Nukleinsäure wird amplifiziert und z.B. mittels einer radioaktiven Markierung identifiziert.

Bei dem aus der WO 95/02702 bekannten Verfahren werden verschiedene Nukleinsäuren in Kombination mit verschiedenen Partikeln verwendet. Damit lässt sich ein komplexer Markierungscode erzeugen. Zur Identifizierung werden die Nukleinsäuren mittels PCR, ggf. in Kombination mit einer Sequenzierung, amplifiziert. Zur Identifizierung des Codes ist weiterhin eine Identifizierung der Partikel und eine Kombination mit den aus den Identifizierungen gewonnenen Informationen erforderlich.

Die US 5,656,731 beschreibt eine Markierung von Antikörpern mittels Nukleinsäuren. Das Vorliegen des gesuchten Antikörpers wird nach einem vorhergehenden Selektionsprozess anhand eines Nachweises der Nukleinsäure vorgenommen. Die Nukleinsäure wird dazu amplifiziert.

Eine Markierung von polymeren Substanzen ist in der US 5,708,153 beschrieben. Dabei wird gleichzeitig mit der Synthese der polymeren Substanz eine die Synthese protokollierende Nukleinsäure synthetisiert. Zur Identifizierung der Zusammensetzung der polymeren Substanz wird die Nukleinsäure amplifiziert und sequenziert.

Die EP 0 794 261 A beschreibt ein Verfahren zur Erkennung und Unterscheidung von verschiedenen Analyten in einer Probe unter Verwendung von zu verschiedenen Zielsequenzen komplementären Nukleinsäuresonden. Die verwendeten Nukleinsäuresonden tragen verschiedene Fluoreszenzmarker und erlauben die Erkennung von Sonden oder Zielsequenzkomplexen.

Die bekannten Verfahren sind kosten- und zeitaufwändig. So weit eine Sequenzierung zur Identifizierung der Markierung erforderlich ist, ist die Benutzung eines komplexen Markierungscodes oder die Identifizierung einer Markierung aus einer Vielzahl von Markierungen nur mit äußersten Schwierigkeiten möglich.

Aufgabe der Erfindung ist es, ein Verfahren anzugeben, mit dem eine Vielzahl von Substanzen eindeutig markiert sowie schnell und kostengünstig identifiziert werden können.

Diese Aufgabe wird duch die Merkmale des Anspruchs 1 gelöst. Zweckmäßige Ausgestaltungen ergeben sich aus den Merkmalen der Ansprüche 2 bis 21.

Nach Maßgabe der Erfindung ist ein Verfahren zur Markierung und Identifizierung von festen, flüssigen und gasförmigen Substanzen vorgesehen, wobei zur Markierung aus einer ersten Gruppe vorgegebener jeweils einen Markierungssequenzabschnitt aufweisender Nukleinsäuresequenzen mindestens eine ausgewählt und zur Substanz hinzugefügt wird,
wobei zur Identifizierung eine zweite Gruppe weiterer Nukleinsäuresequenzen vorgesehen ist, die jeweils einen zu einem der Identifizierungssequenzabschnitte komplementären Nachweissequenzabschhitt aufweisen,
wobei erste Schmelzpunkte von aus den Identifizierungssequenzabschnitten mit den dazu komplementären Nachweissequenzabschnitten gebildete Hybride sich um höchstens 5°C voneinander unterscheiden und
zweite Schmelzpunkte von aus den Identifizierungssequenzabschnitten und Nachweissequenzabschnitten gebildete nicht vollständig komplementäre Hybride um mehr als 5°C niedriger sind als der niedrigste der ersten Schmelzpunkte und
wobei zur Identifizierung die aus der ersten Gruppe ausgewählte/n Nukleinsäuresequenz/en mit den weiteren Nukleinsäuresequenzen der zweiten Gruppe unter vorgegebenen Hybridisierungsbedingungen in Kontakt gebracht und die Hybridisierung nachgewiesen wird.

Das Verfahren kann einfach, schnell und kostengünstig durchgeführt werden. Es ist möglich, sämtliche zur Markierung verwendeten Identifizierungsabschnitte in einem einzigen Reaktionsansatz zu amplifizieren und zu identifizieren.

Unter Nukleinesäuresequnezen werden hier sowohl einzel- als auch doppelsträngige Sequnzen verstanden, die im wesentlichen aus Nukleinsäuren bestehen.

Nach einem Ausgestaltungsmerkmal befindet sich der Identifizierungssequenzabschnitt zwischen zwei Primerbindungssequenzabschnitten. - In diesem Fall kann die Nukleinsäuresequenz einzelsträngig sein. Eine Amplifikation ist beispielsweise mittels der Polymerase-Ketten-Reaktion (PCR) einfach möglich.

Nach einer weiteren Ausgestaltung weisen jeweils zwei Nukleinsäuresequenzen einen Teilabschnitt eines gemeinsamen Identifizierungssequenzabschnitts an ihrem 5'-Ende auf und an den Teilabschnitt ist ein Primerbindungssequenzabschnitt gebunden. Bei dieser Ausgestaltungsform ist der Identifizierungssequenzabschnitt zunächst nicht vorhanden, sondern wird erst während der Amplifizierungsreaktion erzeugt. Vorteilhafterweise sind dabei die Teilabschnitte teilweise komplementär zueinander. - Das erhöht die Sicherheit der Markierung.

Zweckmäßigerweise weisen die Primerbindungssequenzabschnitte den gleichen Schmelzpunkt auf. Das ermöglicht eine gleichzeitige Amplifizierungsreaktion der Nukleinsäuresequenzen in einem einzigen Reaktionsansatz.

Die Nukleinsäuresequenzen können, vorzugsweise mittels PCR und unter Verwendung fluoreszierender Primer, amplifiziert werden. Zur Verbesserung der Stabilität der Markierung ist vorteilhafterweise vorgesehen, daß die Nukleinsäuresequenzen an mindestens einem Ende mit einem Stoff verbunden sind, welcher einem durch Exonuklease bedingten Abbau entgegenwirkt.

Zur Erzeugung komplexer Markierungen können die Nukleinsäuresequenzen mit einer spezifischen Kopplungsgruppe versehen sein. Die Kopplungsgruppe kann aus der folgenden Gruppe ausgewählt sein: Biotin-, Amino-, Thiol-Gruppe oder Hapten. Mittel der Kopplungsgruppe kann die damit versehene Nukleinsäuresequenz spezifisch gebunden und/oder ggf. auch identifiziert werden.

Zur Identifizierung können auch weitere Primerbindungssequenzabschnitte verwendet werden. Jeder Primerbindungssequenzabschnitt kann anhand einer daran gebundenen spezifischen fluorophoren Gruppe eindeutig identifiziert werden.

Zur weiteren Erleichterung der Identifizierung kann an die Nukleinsäuresequenz ein eine fluorophore Gruppe tragendes Molekül gebunden sein.

Insbesondere bei Verwendung identischer Identifikationssequenzabschnitte können zur Identifizierung auch weitere Primerbindungssequenzabschnitte verwendet werden. Jeder der Primerbindungssequenzabschnitte kann anhand einer daran gebundenen spezifischen fluorophoren Gruppe eindeutig identifiziert werden.

Als zweckmäßig hat es sich erwiesen, die Nukleinsäuresequenz an die Substanz zu binden und als Substanz einen der folgenden Stoffe zu verwenden: Antikörper, Lektine, Rezeptoren, Nukleotid-Sequenzen, PNA-Sequenzen, Peptide, Proteine, Zucker, Liganden. Als besonders vorteilhaft wird es angesehen, die Nukleinsäuresequenzen an Partikel zu binden oder darin einzuschließen. Die Partikel können eine Größe von 30 nm bis 3 mm aufweisen. Sie sind vorteilhafterweise Silica-, Polystryol-, Polyvinylchlorid-, Polyethylen, Nylon- oder Glasmilch-Partikel. Das Partikel kann aber auch ein virales Kapsid oder ein virus-like-Partikel sein.- Die Verwendung von Nukleinsäuresequenzen tragenden Partikeln ist besonders vorteilhaft, weil aufgrund der Größe der Partikel eine Sortierung und Isolierung beispielsweise in einem Partikel-Sortierer möglich ist. Das Partikel kann als Träger einer Markierung an die zu markierende Substanz gebunden sein.

Zur Identifizierung hat es sich als vorteilhaft erwiesen, daß jede der weiteren Nukleinsäuresequenzen an einem.vorgegebenen Ort einer festen Oberfläche, vorzugsweise auf einem Chip, einer Mikrotiterplatte oder Folie, gebunden ist. Das ermöglicht eine Entschlüsselung komplexer Markierungscodes oder einer Vielzahl von Codes in einem einzigen Verfahrensschritt.

Die Hybridisierung eines Identifizierungssequenzabschnitts mit einem komplementären Nachweissequenzabschnitt kann mittels Fluoreszenz nachgewiesen werden. Die vorgeschlagene Identifizierung ist besonders einfach erkennbar.

Nach einer weiteren Ausgestaltung ist vorgesehen, daß mindestens zwei Nukleinsäuresequenzen als Markierung der Substanz zugefügt werden. Dadurch kann mit einer geringen Anzahl unterschiedlicher Identifizierungssequenzabschnitte ein komplexer Code bereitgestellt werden.

Die Nukleinsäuresequenzen und/oder die weiteren Nukleinsäuresequenzen werden vorzugsweise synthetisch hergestellt.

Anstatt der Nukleinsäuresequenzen bzw. der weiteren Nukleinsäuresequenzen können auch Chimäre aus Nukleinsäuren und Nukleinsäureanaloga, wie PTO oder PNA, verwendet werden. Solche Chimäre weisen eine erhöhte Stabilität gegenüber einem enzymathischen Abbau auf.

Nachfolgend werden anhand der Zeichnung Ausführungsbeispiele der Erfindung näher erläutert. Es zeigen:
- Fig. 1 a - e: die Amplifikation einer ersten Nukleinsäuresequenz,
- Fig. 2 a - e: die Amplifikation zweier zweiter Nukleinsäuresequenzen,
- Fig. 3 a - d: die Identifikation einer ersten Nukleinsäuresequenz mittels "molecular beacons",
- Fig. 4: die Markierung eines Identifizierungssequenzabschnitts,
- Fig. 5 a: mit fluorphoren Gruppen markierte Nukleinsäuresequenzen,

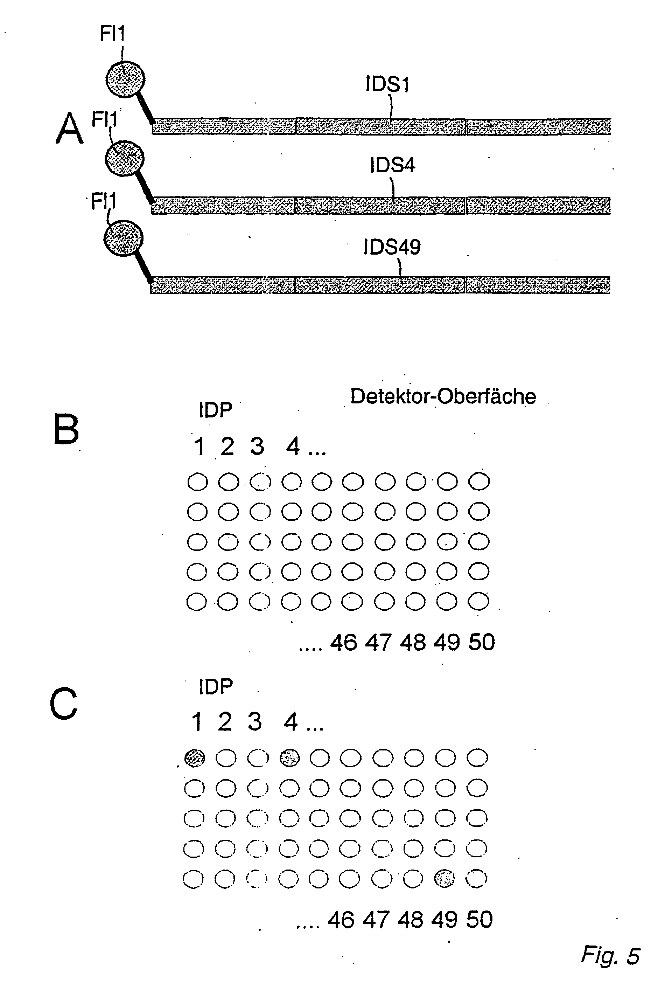

- Fig. 5 b, c: einen Detektor mit den weiteren Nukleinsäuresequenzen,

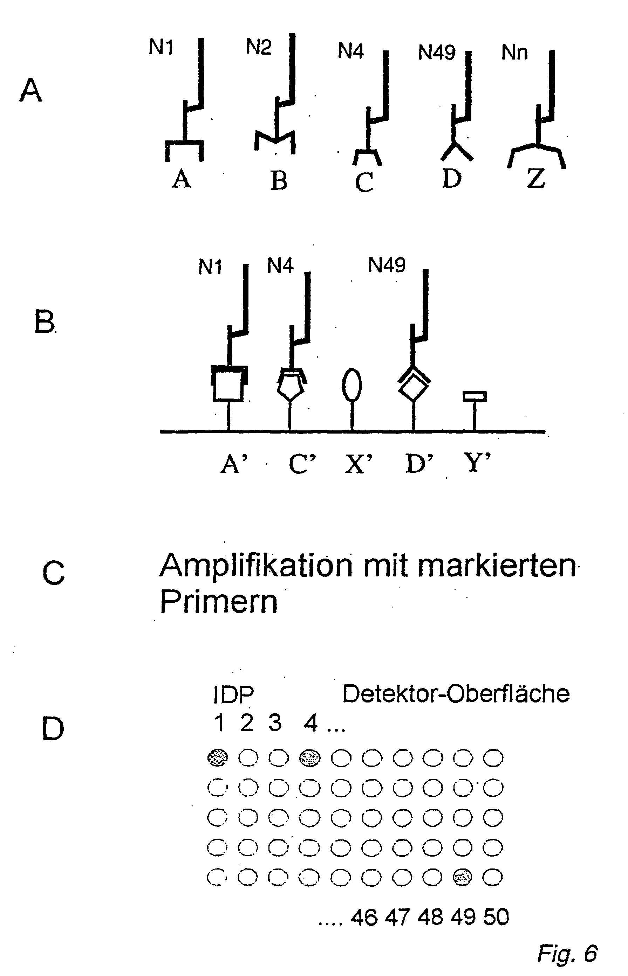

- Fig. 6 a - d: die Selektion und Identifizierung dritter Nukleinsäuresequenzen,

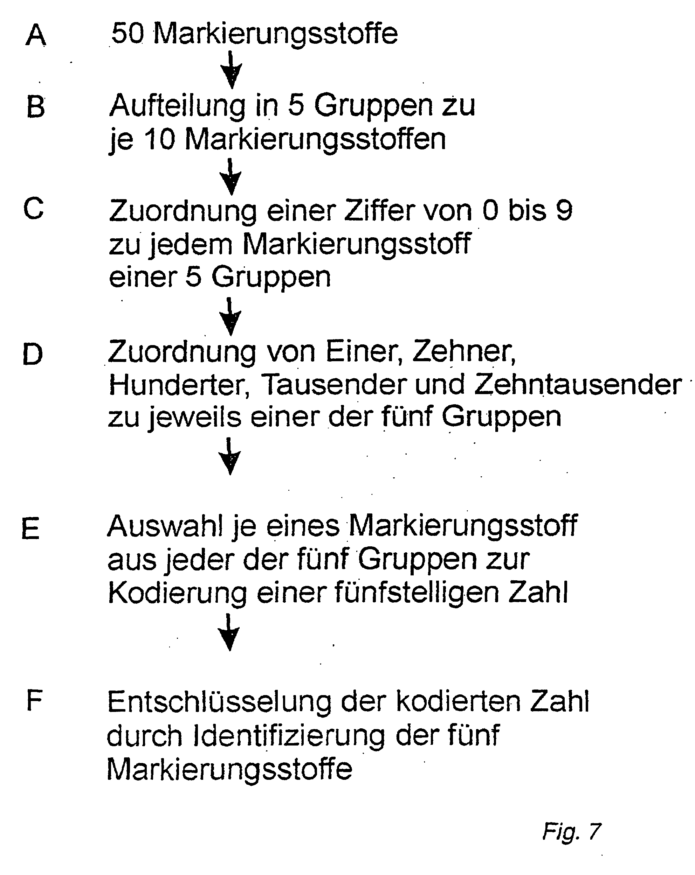

- Fig. 7 a - f: die Herstellung eines komplexen Codes,

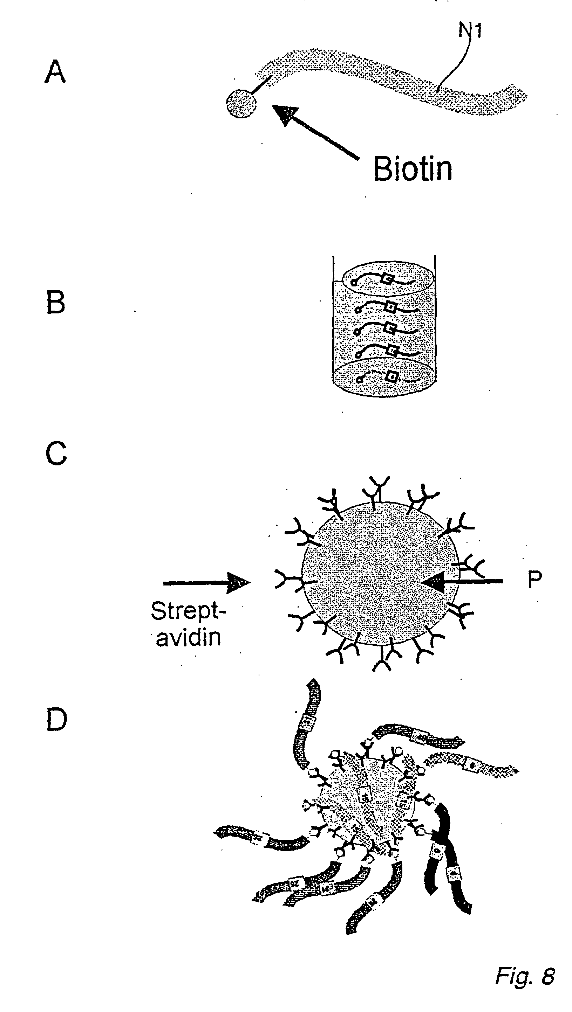

- Fig. 8 a - d: die Herstellung von Markierungs-Partikeln,

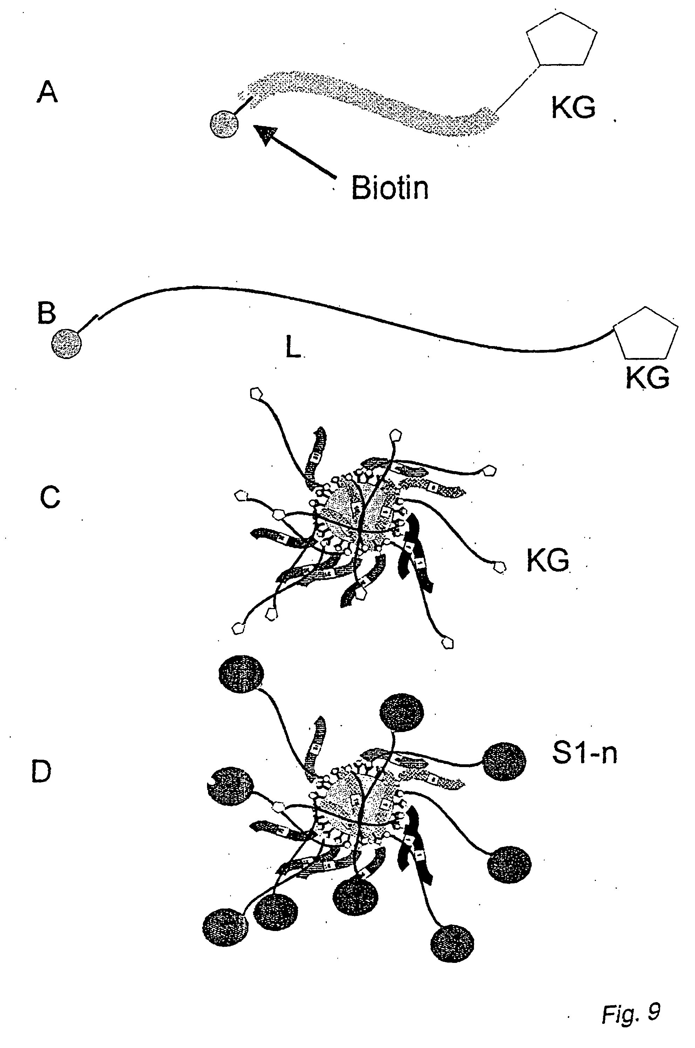

- Fig. 9 a - d: die Markierung von Molekülen mit Markierungs-Partikeln gemäß Fig.8 und

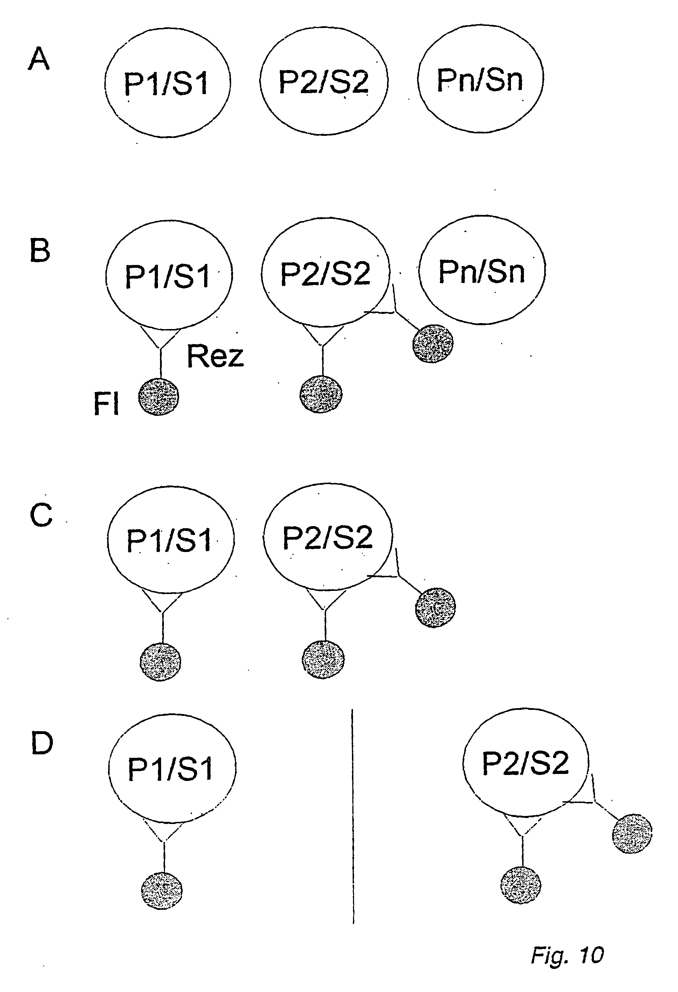

- Fig. 10: die Selektion und Ideritifizierung von mit Markierungs-Partikeln markierten Substanzen.

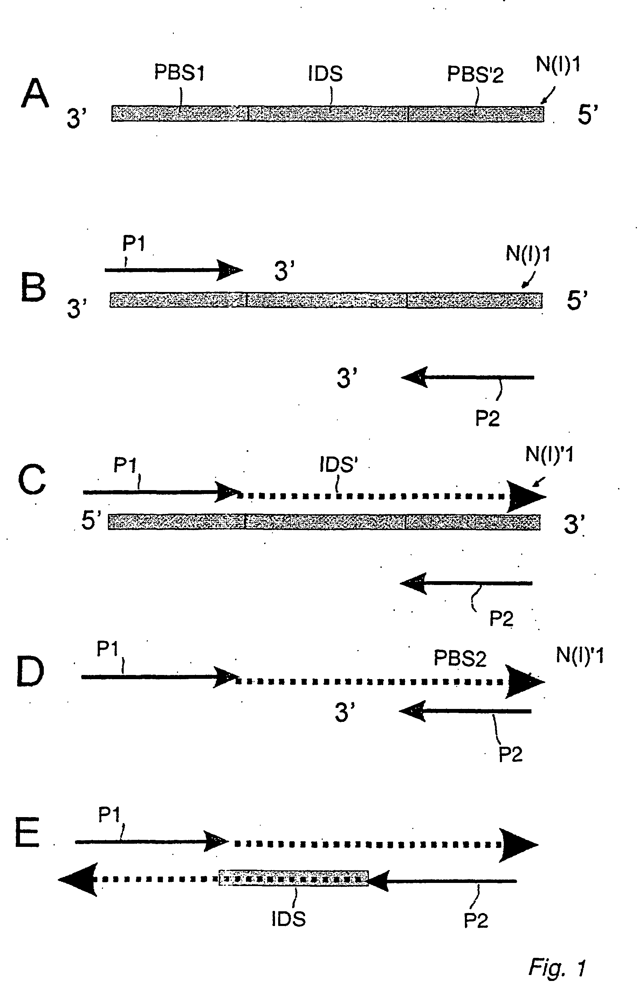

In den Fig. 1 a - e ist eine erste Nukleinsäuresequenz N(I)1 und deren Amplifikation schematisch dargestellt. Die erste Nukleinsäuresequenz N(I)1 weist an ihrem 3'-Ende einen ersten Primerbindungssequenzabschnitt PBS1 und an ihrem 5'-Ende einen zu einem zweiten (hier nicht gezeigten) Primerbindungssequenzabschnitt PBS2 komplementären zweiten Primerbindungssequenzabschnitt PBS'2 auf. Zwischen dem ersten PBS1 und dem zweiten komplementären Primerbindungssequenzabschnitt PBS'2 befindet sich ein Identifizierungsequenzabschnitt IDS.

Zur Amplifikation wird die erste Nukleinsäuresequenz N(I)1 mit einem ersten Primer P1 und einem zweiten Primer P2 in Kontakt gebracht. Der erste P1 und der zweite Primer P2 hybridisieren mit dem dazu komplementären ersten Primerbindungssequenzabschnitt PBS1 bzw. zweiten Primerbindungssequenzabschnitt PBS2. Durch Polymerase werden der erste P1 und der zweite Primer P2 verlängert; es wird ein zum Identifizierungssequenzabschnitt IDS komplementärer Identifizierungssequenzabschnitt IDS' gebildet (Fig. 1c). Der zweite Primer P2 bindet dann an die komplementäre Nukleinsäuresequenz N'(I)1 (Fig.1d). Durch die Polymerase wird nun eine doppelsträngige DNA gebildet, welche den Identifizierungssequenzabschnitt IDS enthält (Fig. 1e).

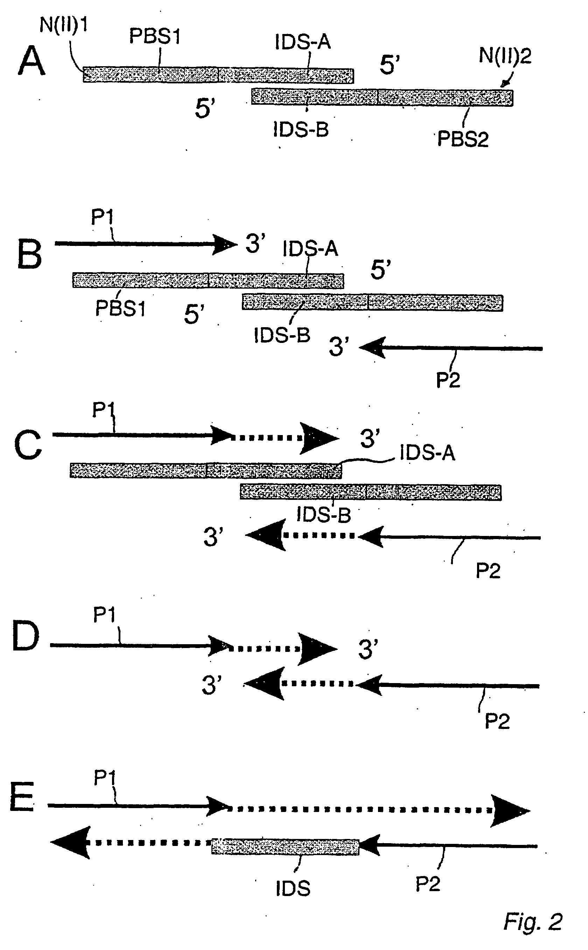

Bei dem in den Fig.2 a - e gezeigten Ausführungsbeispiel ist der Identifizierungssequenzabschnitt IDS zunächst nicht vorhanden. Eine zweite Nukleinsäuresequenz N(II)1 weist an ihrem 5'-Ende den ersten Primerbindungssequenzabschnitt PBS1 auf. Daran gebunden ist ein Teilabschnitt IDS-A des gemeinsamen Identifizierungsequenzabschnitts IDS. Eine weitere zweite Nukleinsäuresequenz N(II)2 weist an ihrem 3'-Ende den zweiten Primerbindungssequenzabschnitt PBS2 auf. Daran ist ein zweiter Teilabschnitt IDS-B des Identifikationssequenzabschnitts IDS gebunden. Der erste Teilabschnitt IDS-A und der zweite Teilabschnitt IDS-B sind abschnittsweise komplemtär zueinander.

Die Amplifikation ist in den Fig. 2b - e gezeigt. Der erste Primer P1 und der zweite Primer P2 binden an die dazu jeweils komplementären Primersequenzabschnitte PBS1 bzw. PBS2. Durch Polymerase werden die komplementären Sequenzen des ersten IDS-A und des zweiten Teilabschnitts IDS-B synthetisiert (Fig. 2c). Diese Syntheseprodukte können in weiteren Zyklen mit ihren 3'-Enden hybridisieren (Fig.2d) und verlängert werden (Fig. 2e). Es entsteht als Produkt eine Nukleinsäuresequenz, welche den vollständigen Identifizierungssequenzabschnitt IDS aufweist.

Bei dieser Verfahrensvariante können vorteilhafterweise im Bereich des ersten PBS1 und zweiten Primerbindungssequenzabschnitts PBS2 Nukleinsäure-Analoga, wie z.B. PNA oder PTO, verwendet werden. Solche Nukleinsäure-Analoga weisen eine erhöhte Stabilität gegenüber einem enzymatischen Abbau auf. Zur weiteren Erhöhung der Stabilität kann auch das 5'-Ende der Nukleinsäuresequenz mit einem Stoff gekoppelt werden, der einen 5'-Exonuklease-Abbau verhindert. Als Stoffe eignen sich dazu z.B. PNA oder PTO.

Die Primerbindungssequenzabschnitte PBS1, PBS2 sind vorteilhafterweise so gewählt, daß die Amplifikationsreaktion in einem engen Temperaturbereich durchgeführt werden kann. Dazu werden die Primerbindungssequenzabschnitte PBS1 bzw. PBS2 so gewählt, daß sich ihre Schmelzpunkte um höchstens 5° Celsius voneinander unterscheiden. Um die Spezifität der Identifizierungsreaktion zu erhöhen ist es vorteilhaft, daß der Schmelzpunkt der Primerbindungssequenzabschnitte PBS1 bzw. PBS2 bei einer Bildung von nicht vollständig komplementären Hybriden um mehr als 5° Celsius unterhalb des niedrigsten Schmelzpunkts eines vollständig komplementären Hybrids liegt. Dadurch wird die Bildung unspezifischer Hybride während der Amplifizierung unmöglich gemacht.

Zur Identifizierung der Markierung wird der Identifizierungssequenzabschnitt IDS mit einem dazu vollständig komplementären Nachweissequenzabschnitt hybridisiert. Um die Spezifität der Identifizierungsreaktion zu erhöhen ist vorgesehen, daß sämtliche Identifizierungssequenzabschnitte IDS mit den dazu komplementären Nachweissequenzabschnitten IDP erste Schmelzpunkte aufweisen, die sich vorzugsweise um nicht mehr als 5° Celsius voneinander unterscheiden. Zur weiteren Erhöhung der Spezifität ist vorgesehen, daß jeder Schmelzpunkt eines nicht vollständigen Hybrids mit einem Identifizierungssequenzabschnitt IDS mehr als 5° Celsius unterhalb des niedrigsten Schmelzpunkts vollständig komplementärer Hybride liegt.

Zur weiteren Vereinfachung der Verfahrensführung ist außerdem vorgesehen, daß die Schmelzpunkte der Primerbindungssequenzabschnitte PBS1, PBS2 und der Identifizierungssequenzabschnitte IDS im wesentlichen gleich sind.

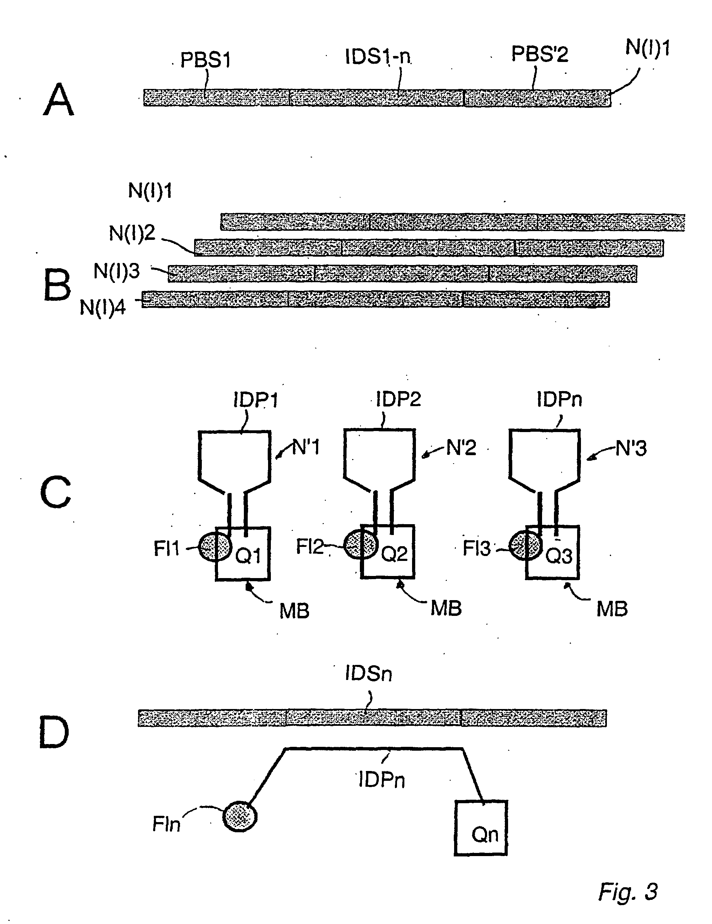

Nach einer weiteren Verfahrensvariante wird eine erste Nukleinsäuresequenz N(I)1 in einer Amplifizierungsreaktion vervielfältigt (Fig. 3a und b). Im Reaktionsansatz befinden sich außerdem molecular beacons, die zu den Identifizierungssequenzabschnitten komplementäre Nachweissequenzabschnitte IDP1-n aufweisen. Die molecular beacons MB sind in Form einer Haarnadelschleife ausgebildet. Im Bereich der Enden der molecular beacons befinden sich eine fluorophore Gruppe F11, F12, F13 sowie in gegenüberliegender Anordnung ein Quencher Q1, Q2, Q3.

Sobald eine ausreichende Anzahl erster Nukleinsäuresequenzen N(I)1-n durch Amplifikation hergestellt worden ist, kommt es zur spezifischen Hybridisierung des Identifizierungssequenzabschnitts IDS1-n mit dem dazu komplementären Nachweissequenzabschnitt IDP1-n. Dabei wird die räumliche Beziehung zwischen dem Quencher Q1, Q2, Q3 und der fluorophoren Gruppe F11, F12, F13 aufgehoben. Eine spezifische Fluoreszenz ist beobächtbar (Fig. 3d).

Durch die Verwendung verschiedener Fluorophore F11, F12, F13 in den molecular beacons MB ist eine Unterscheidung von verschiedenen Identifizierungssequenzabschnitten IDS1-n möglich.

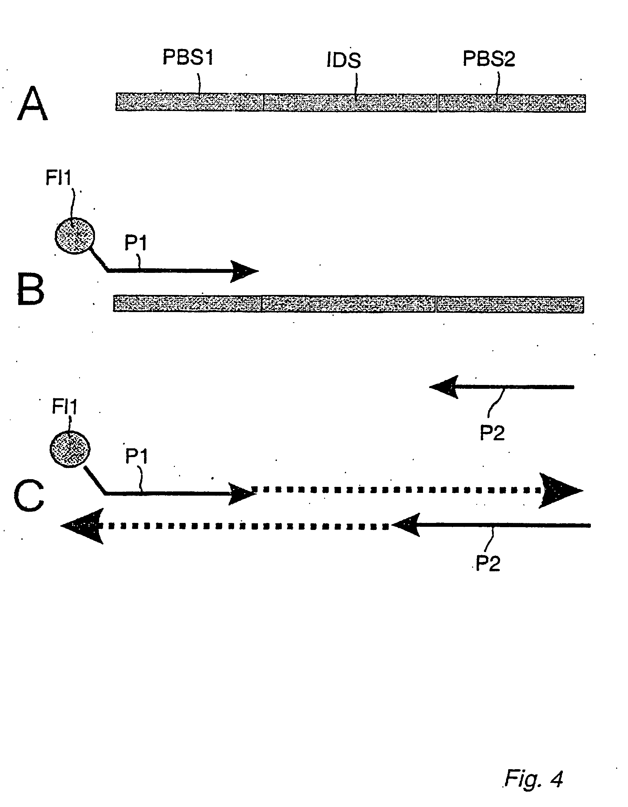

Bei der in Fig.4a - c gezeigten Verfahrensvariante wird die Amplifikation mit einem ersten Primer P1 durchgeführt, der mit einer fluorophoren Gruppe F11 markiert ist. Bei der Verwendung von Nukleinsäuresequnezen mit identischem Identifikationssequnenzabschnitt IDS kann eine Unterscheidung durch die Benutzung unterschiedlicher Primerbindungssequenzabschnitte erfolgen, die jeweils mit spezifischen fluorophoren Gruppen markiert sind.

Zur Identifikation des Identifikationssequenzabschnitts IDS wird die bei der Amplifikation mit der fluorophoren Gruppe F11 hergestellte Nukleinsäuresequenz mit einer komplementären Nachweisnukleotisequenz IDP in Kontakt gebracht, die an einem vorgegebenen Ort einer festen Oberfläche gebundenen ist. Die dann an dem vorgegebenen Ort auftretende Fluoreszenz kann mittels eines herkömmlichen Nachweisgeräts erfaßt werden (siehe Fig. 5a - c).

Nach einer weiteren Verfahrensvariante sind die Nukleinsäuresequenzen N1-n mit Kopplungsgruppen verbunden, die eine Bindung an weitere Substanzen ermöglichen. Zu diesen Kopplungsgruppen gehören Biotin, Aminolinker, Thiolgruppen oder Haptene, wie Digoxigenin. Mit diesen Kopplungsgruppen kann die Nukleinsäuresequenz N1-n an spezifisch zu markierende Moleküle gebunden und/ oder markiert werden.

Es können mittels der allgemein mit KG bezeichneten Kopplungsgruppen unterschiedliche Antikörper mit der erfindungsgemäßen Nukleinsäuresequenz N1-n eindeutig markiert werden. Die Bindung kann über ein ProteinA/Streptavidin-Fusionsprotein vermittelt werden. Dieses Protein bindet die konstante Region von Antikörpern und vermittelt eine Affinität zu Biotin. Biotinylierte Nukleinsäuresequenzen binden an solche Antikörper. Damit ist eine Selektion möglich. In den Fig. 6 a - d ist eine solche Selektion gezeigt. Antikörper A, B, C, D und Z sind mit den Nukleinsäuresequenzen N1, N2, N4, N49 und Nn markiert. Die Antikörper A, B, C, D und Z werden mit einer Matrix in Kontakt gebracht, auf der verschiedene Antigene A', C', X', D' und Y' fixiert sind. Die Antikörper A, C, D, deren Antigen A', C' und D'auf der Matrix fixiert ist, werden spezifisch gebunden (Fig. 6b). Nach dem Entfernen der nicht an die Matrix gebundenen Antikörper B, Z durch Waschen können die Nukleinsäuresequenzen N1, N4 und N49 mit markierten Primern amplifiziert werden (Fig. 6c bzw. Fig. 5a). Die amplifizierten Nukleinsäuresequenzen N1, N4, N49 werden mit einer Detektor-Oberfläche in Kontakt gebracht, auf der die die Nachweissequenzabschnitte IDP enthaltenden weiteren Nukleinsäuresequenzen N1-n gebunden sind. Dabei nimmt jede weitere Nukleinsäuresequenz N'1-n eine bestimmte vorgegebene Position auf der Detektor-Oberfläche ein (siehe Fig. 6d).

In Fig.7a - f ist in Form eines Flußdiagramms die Herstellung und Identifizierung eines komplexen Codes gezeigt. Es werden 50 verschiedene Nukleinsäuresequenzen benutzt. Die 50 Nukleinsäuresequenzen werden in 5 Gruppen zu je 10 Nukleinsäuresequenzen unterteilt (Fig. 7b). Jeder Nukleinsäuresequenz einer Gruppe wird eine Ziffer von 0 bis 9 zugeordnet (Fig. 7c). Jeder Gruppe von Nukleinsäuresequenzen wird eine Stelle einer 5 stelligen Zahl zugeordnet (Fig..7d).

Zur Herstellung des Codes wird jeder Gruppe der 5 Gruppen genau eine Nukleinsäuresequenz entnommen und diese zur Markierung verwendet (Fig. 7e). Jede Mischung aus den entnommenen 5 Nukleinsäuresequenzen definiert somit eine Zahl zwischen 0 und 99999. Die Identifikation des Zahlenwerts erfolgt duch die Identifikation der 5 Nukleinsäuresequenzen (Fig. 7f).

Nach einer weiteren Ausführungsvariante des Verfahrens können die Nukleinsäuresequenzen N1-n an Partikel P gebunden werden. Die Bindung erfolgt vorzugsweise über aktivierte oder aktivierbare Gruppen. Zu diesen Gruppen gehören Biotin, Aminolinker, Thiolgruppen oder Haptene, wie Digoxigenin. Als Partikel P können Polystryrol-, Silica-, Polyvinylchlorid-, Polyethylen-, Nylon oder Glasmilch-Partikel verwendet werden. Auch aus Virushüllen oder virus-like-particles bestehende Partikel können Verwendung finden. Die Partikel P können auch aus Stoffen hergestellt sein, die DNA komplexieren, wie z.B. Polylysin oder DNA-bindende Proteine.

Die Partikel P können mit mehreren Nukleinsäuresequenzen N1-n markiert sein. Ein Partikel P kann somit einen Zahlencode nach dem vorgenannten Beispiel tragen. In den Fig. 8a-d ist die Herstellung eines markierten Partikels P beispielhaft dargestellt. Die Nukleinsäuresequenz N1-n trägt an ihrem einen Ende eine Biotingruppe (Fig. 8a). Verschiedene solcher biotinyliarten Nukleinsäuresequenzen werden im gleichen molaren Verhältnis gelöst (Fig. 8b). Zur Lösung wird eine vorgegebene Menge an Partikeln P gegeben, die beispielsweise mit Streptavidin beschichtet sind (Fig. 8c). Es bildet sich zwischen Biotin und Streptavidin eine Bindung. Die Nukleinsäuresequenzen N1-n werden so auf dem Partikel P gebunden (Fig. 8d). Es können auf dem Partikel P selbstverständlich mehrere Nukleinsäuresequenzen N1-n des gleichen Typs gebunden sein. Das erhöht die Reaktionssicherheit bei der Amplifikation.

Die Partikel P besitzen vorzugsweise eine Größe von 1 µm bis 100 µm. Sie können fluoreszierend oder durch eine Bindungsreaktion mit anderen Stoffen fluoreszenzmarkiert sein. Durch ihre Größe und fluoreszente Eigenschaft können die Partikel P mittels eines Partikel-Sortierers sortiert und isoliert werden. Das ermöglicht die Identifizierung von Numerierungen einzelner Partikel P, die Teil einer Mischung mehrerer Partikel P sind.

Nach einer weiteren Ausgestaltung der Erfindung können auch die Partikel P Kopplungsgruppen KG aufweisen, die zur Bindung an die zu markierende Substanz geeignet ist (Fig. 9a). Zu diesen Gruppen gehören Biotin-, Aminolinker-oder Thiolgruoppen. Sie können beispielsweise am Ende der Nukleinsäuresequenz N1-n angebracht sein. Die Kopplungsgruppen KG werden an den Partikel vorzugsweise über Spacermoleküle L gebunden (Fig. 9b).

Fig. 9c zeigt ein Partikel P mit freien Kopplungsgruppen KG. Fig. 9d zeigt ein Partikel P, bei dem Substanz S an die Kopplungsgruppen KG gebunden ist.

Die Substanz S1-n kann beispielsweise mit einem potentiellen Rezeptor R umgesetzt werden (Fig. 10a). Der Rezeptor R kann mit einem fluorophoren Molekül markiert sein (Fig. 10b). Partikel P, die einen Liganden des Rezeptors R tragen, werden vom Rezeptor R gebunden. Die den Rezeptor R aufweisenden Partikel P können aufgrund ihrer Größe und Fluoreszenz mittels eines Fluoreszenz-aktivierten Partikel-Sortierers von Partikeln P getrennt werden, die keinen Rezeptor R gebunden haben (Fig. 10c). Die Identifizierung der gebunden Substanz kann anhand des Identifikationssequenzabschnitts IDS der Nukleinsäuresequenz N1-n erfolgen.

### Bezugszeichenliste

- N1-n: Nukleinsäuresequenz
- N'1-n: weitere Nukleinsäuresequenz
- IDS1-n: Identifizierungssequenzabschnitt
- IDP1-n: Nachweissequenzabschnitt
- IDS-A, IDS-B: Teilabschnitt
- PBS1: erster Primerbindungssequenzabschnitt
- PBS2: zweiter Primerbindungssequenzabschnitt
- KG,A,B,C,D,Z: Kopplungsgruppen.
- L: Spacermolekül
- S: Substanz
- R: Rezeptor
- F11: fluorophore Gruppe
- P: Partikel

## Patentansprüche

1. Verfahren zur Markierung und Identifizierung von festen, flüssigen und gasförmigen Substanzen (S1-n),
wobei zur Markierung aus einer ersten Gruppe vorgegebener jeweils einen Identifizierungssequenzabschnitt (IDS1-n) aufweisenden Nukleinsäuresequenzen (N1-n) mindestens eine ausgewählt und zur Substanz (S1-n) hinzugefügt wird,
wobei zur Identifizierung eine zweite Gruppe weiterer Nukleinsäuresequenzen (N'1-n) vorgesehen ist, die jeweils einen zu einem der Identifizierungssequenzabschnitte (IDS1-n) komplementären Nachweissequenzabschnitt (IDP1-n) aufweisen,
wobei erste Schmelzpunkte von aus den Identifizierurigssequenzabschnitten (IDS1-n) mit den dazu komplementären Nachweissequenzabschnitteh (IDP1-n) gebildeten Hybriden sich um höchstens 5°C voneinander unterscheiden und
zweite Schmelzpunkte von aus den Identifizierungssequenzabschnitten (IDS1-n) und Nachweissequenzabschnitten (IDP1-n) gebildeten nicht vollständig komplementären Hybriden um mehr als 5°C niedriger sind als der niedrigste der ersten Schmelzpunkte und
wobei zur Identifizierung die aus der ersten Gruppe ausgewählte/n Nukleinsäuresequenz/en (N1-n) mit den weiteren Nukleinsäuresequenzen (N'1-n) der zweiten Gruppe unter vorgegebenen Hybridisierungsbedingungen in Kontakt gebracht und die Hybridisierung nachgewiesen wird.

2. Verfahren nach Anspruch 1, wobei der Identifizierungssequenzabschnitt (IDS1-n) zwischen zwei Primerbindungsequenzabschnitten (PBS1, PBS2) sich befindet.

3. Verfahren nach Anspruch 1 oder 2, wobei jeweils zwei Nukleinsäuresequenzen (N1-n) einen Teilabschnitt (IDS-A, IDS-B) eines gemeinsamen Identifizierungssequenzabschnitts (IDS1-n) an ihrem 5'-Ende aufweisen und an den Teilabschnitt (IDS-A, IDS-B) ein Primerbindungssequenzabschnitt gebunden ist.

4. Verfahren nach Anspruch 3, wobei die Teilabschnitte (IDS-A, IDS-B) teilweise komplementär zueinander sind.

5. Verfahren nach einem der vorhergehenden Ansprüche, wobei die Primerbindungssequenzabschnitte (PBS1, PBS2) den gleichen Schmelzpunkt aufweisen.

6. Verfahren nach einem der vorhergehenden Ansprüche, wobei die Nukleinsäuresequenzen (N1-n), vorzugsweise mittels PCR und unter Verwendung fluoreszierender Primer, amplifiziert werden.

7. Verfahren nach einem der vorhergehenden Ansprüche, wobei die Nukleinsäuresequenzen (N1-n) an mindestens einem Ende mit einem Stoff verbunden sind, welcher einem durch Exonuklease bedingten Abbau entgegenwirkt.

8. Verfahren nach einem der vorhergehenden Ansprüche, wobei die Nukleinsäuresequenz (N1-n) mit einer Kopplungsgruppe versehen ist.

9. Verfahren nach einem der vorhergehenden Ansprüche, wobei die Kopplungsgruppe aus der folgenden Gruppe ausgewählt ist: Biotin-, Amino-, Thiol-Gruppe oder Hapten.

10. Verfahren nach einem der vorhergehenden Ansprüche, wobei an die Nukleinsäuresequenz (N1-n) ein eine fluorophore Gruppe (F11-n) tragendes Molekül gebunden ist.

11. Verfahren nach einem der vorhergehenden Ansprüche, wobei die Kopplungsgruppe mit einer fluorophoren Gruppe markiert ist.

12. Verfahren nach einem der vorhergehenden Ansprüche, wobei die Nukleinsäuresequenzen (N1-n) an die Substanz (S1-n) gebunden und als Substanz (S1-n) einer der folgenden Stoffe verwendet wird: Antikörper, Lektine, Rezptoren, Nukleotid-Sequenzen, PNA-Sequenzen, Peptide, Proteine, Zucker, Liganden.

13. Verfahren nach einem der vorhergehenden Ansprüche, wobei die Nukleinsäuresequenzen (N1-n) an Partikel (P) gebunden sind.

14. Verfahren nach einem der vorhergehenden Ansprüche, wobei die Partikel (P) eine Größe von 30 nm bis 3 mm aufweisen.

15. Verfahren nach einem der vorhergehenden Ansprüche, wobei die Partikel (P) Silica-, Polystyrol-, Polyvinylchlorid-, Polyethylen, Nylon- oder Glasmilch-Partikel sind.

16. Verfahren nach einem der vorhergehenden Ansprüche, wobei das Partikel (P) ein virales Kapsid oder ein virus-likeparticle ist.

17. Verfahren nach einem der vorhergehenden Ansprüche, wobei jede der weiteren Nukleinsäuresequenzen (N'1-n) an einem vorgegebenen Ort einer festen Oberfläche, vorzugsweise auf einem Chip, einer Mikrotiterplatte oder Folie, gebunden ist.

18. Verfahren nach einem der vorhergehenden Ansprüche, wobei die Hybridisierung eines Identifizierungssequenzabschnitts (IDS1-n) mit einem komplementären Nachweissequenzabschnitt (IDP1-n) mittels Fluoreszenz nachgewiesen wird.

19. Verfahren nach einem der vorhergehenden Ansprüche, wobei mindestens zwei Nukleinsäuresequenzen (N1-n) als Markierung der Substanz (S1-n) zugefügt werden.

20. Verfahren nach einem der vorhergehenden Ansprüche, wobei die Nukleinsäuresequenzen (N1-n) und/oder die weiteren Nukleinsäuresequenzen (N'1-n) synthetisch hergestellt werden.

21. Verfahren nach einem der vorhergehenden Ansprüche, wobei anstatt der Nukleinsäuresequenzen bzw. der weiteren Nukleinsäuresequenzen Chimäre aus Nukleinsäuren und Nukleinsäureanaloga, wie PTO oder PNA, verwendet werden.

## Claims

1. A method for labeling and identifying solid, liquid and gaseous substances (S1-n),
wherein labeling is carried out by selecting at least one nucleic acid sequence from a first group of predefined nucleic acid sequences (N1-n) having in each case an identification sequence section (IDS1-n) and adding it to the substance (S1-n),
wherein a second group of further nucleic acid sequences (N'1-n) which have in each case a detection sequence section (IDP1-n) complementary to one of the identification sequence sections (IDS1-n) is provided for identification,
wherein first melting points of hybrids formed from the identification sequence sections (IDS1-n) together with the detection sequence sections (IDP1-n) complementary thereto differ by not more than 5°C from one another and
second melting points of not completely complementary hybrids formed from the identification sequence sections (IDS1-n) and detection sequence sections (IDP1-n) are more than 5°C lower than the lowest of the first melting points and
wherein identification is carried out by contacting the nucleic acid sequence(s) (N1-n) selected from the first group with the further nucleic acid sequences (N'1-n) of the second group under predefined hybridization conditions and detecting hybridization.

2. The method as claimed in claim 1, wherein the identification sequence section (IDS1-n) is located between two primer binding sequence sections (PBS1, PBS2).

3. The method as claimed in claim 1 or 2, wherein in each case two nucleic acid sequences (N1-n) have a part section (IDS-A, IDS-B) of a common identification sequence section (IDS1-n) at their 5' end and a primer binding sequence section is bound to said part section (IDS-A, IDS-B).

4. The method as claimed in claim 3, wherein the part sections (IDS-A, IDS-B) are partly complementary to one another.

5. The method as claimed in any of the preceding claims, wherein the primer binding sequence sections (PBS1, PBS2) have the same melting point.

6. The method as claimed in any of the preceding claims, wherein the nucleic acid sequences (N1-n) are amplified, preferably by means of PCR and by using fluorescent primers.

7. The method as claimed in any of the preceding claims, wherein the nucleic acid sequences (N1-n) are linked on at least one end to an agent which counteracts degradation caused by exonuclease.

8. The method as claimed in any of the preceding claims, wherein the nucleic acid sequence (N1-n) is provided with a coupling group.

9. The method as claimed in any of the preceding claims, wherein the coupling group is selected from the following group: biotin group, amino group, thiol group or hapten.

10. The method as claimed in any of the preceding claims, wherein a molecule carrying a fluorophoric group (F11-n) is bound to the nucleic acid sequence (N1-n).

11. The method as claimed in any of the preceding claims, wherein the coupling group is labeled with a fluorophoric group.

12. The method as claimed in any of the preceding claims, wherein the nucleic acid sequences (N1-n) are bound to the substance (S1-n) and the substance (S1-n) used is one of the following agents: antibodies, lectins, receptors, nucleotide sequences, PNA sequences, peptides, proteins, sugars, ligands.

13. The method as claimed in any of the preceding claims, wherein the nucleic acid sequences (N1-n) are bound to particles (P).

14. The method as claimed in any of the preceding claims, wherein the particles (P) are from 30 nm to 3 mm in size.

15. The method as claimed in any of the preceding claims, wherein the particles (P) are silica, polystyrene, polyvinyl chloride, polyethylene, nylon or glass milk particles.

16. The method as claimed in any of the preceding claims, wherein the particle (P) is a viral capsid or a virus-like particle.

17. The method as claimed in any of the preceding claims, wherein each of the further nucleic acid sequences (N'1-n) is bound to a predefined site on a solid surface, preferably on a chip, a microtiter plate or film.

18. The method as claimed in any of the preceding claims, wherein hybridization of an identification sequence section (IDS1-n) with a complementary detection sequence section (IDP1-n) is detected by means of fluorescence.

19. The method as claimed in any of the preceding claims, wherein at least two nucleic acid sequences (N1-n) are added to the substance (S1-n) as a label.

20. The method as claimed in any of the preceding claims, wherein the nucleic acid sequences (N1-n) and/or the further nucleic acid sequences (N'1-n) are prepared synthetically.

21. The method as claimed in any of the preceding claims, wherein chimeras of nucleic acids and nucleic acid analogs, such as PTO or PNA, are used instead of the nucleic acid sequences or the further nucleic acid sequences.

## Revendications

1. Procédé pour le marquage et l'identification de substances solides, liquides et gazeuses (S1-n),
pour le marquage à partir d'un premier groupe de séquences d'acide nucléique (N1-n), présentant respectivement une section de séquence d'identification (IDS1-n), au moins une séquence étant sélectionnée et ajoutée à la substance (S1-n),
pour l'identification, un deuxième groupe d'autres séquences d'acide nucléique (N'1-n) étant prévu, ces séquences présentant respectivement une section de séquence de détection (IDP1-n) complémentaire à une des sections de séquence d'identification (IDS1-n),
des premiers points de fusion d'hybrides formés à partir des sections de séquence d'identification (IDS1-n) avec les sections de séquence de détection (IDP1-n) complémentaires différant les uns des autres de maximum 5°C et
des seconds points de fusion d'hybrides non entièrement complémentaires formés à partir des sections de séquence d'identification (IDS1-n) et des sections de séquence de détection (IDP1-n) étant inférieurs de plus de 5°C que le plus bas des premiers points de fusion et
pour l'identification, la/les séquence(s) d'acide nucléique (N1-n) sélectionnée(s) à partir du premier groupe entrant en contact dans des conditions d'hybridation définies avec les autres séquences d'acide nucléique (N'1-n) du deuxième groupe et l'hybridation étant identifiée.

2. Procédé selon la revendication 1, la section de séquence d'identification (ISD1-n) se trouvant entre deux sections de séquence de liaison de primer (PBS1, PBS2).

3. Procédé selon la revendication 1 ou 2, respectivement deux séquences d'acide nucléique (N1-n) présentant à son extrémité 5' une section partielle (IDS-A, IDS-B) d'une section de séquence d'identification commune (IDS1-n) et une section de séquence de liaison de primer étant liée à la section partielle (IDS-A, IDS-B).

4. Procédé selon la revendication 3, les sections partielles (IDS-A, IDS-B) étant en partie complémentaires.

5. Procédé selon l'une des revendications précédentes, les sections de séquence de liaison de primer (PBS1, PBS2) présentant le même point de fusion.

6. Procédé selon l'une des revendications précédentes, les séquences d'acide nucléiques (N1-n) étant amplifiées de préférence au moyen d'une PCR et en utilisant des primers fluorescents.

7. Procédé selon l'une des revendications précédentes, les séquences d'acide nucléique (N1-n) étant liées à au moins une extrémité à une substance laquelle s'oppose à une décomposition par exonucléase.

8. Procédé selon l'une des revendications précédentes, la séquence d'acide nucléique (N1-n) étant munie d'un groupe de couplage.

9. Procédé selon l'une des revendications précédentes, le groupe de couplage étant sélectionné à partir du groupe suivant : groupe biotine, groupe amine, groupe thiol ou haptène.

10. Procédé selon l'une des revendications précédentes, une molécule portant un groupe fluorophore (F11-n) étant liée à la séquence d'acide nucléique (N1-n).

11. Procédé selon l'une des revendications précédentes, le groupe de couplage étant marqué avec un groupe fluorophore.

12. Procédé selon l'une des revendications précédentes, les séquences d'acide nucléique (N1-n) étant liées à la substance (S1-n) et utilisées comme substance (S1-n) d'un des corps suivants : anticorps, lectines, récepteurs, séquences nucléotides, séquences PNA, peptides, protéines, sucres, ligands.

13. Procédé selon l'une des revendications précédentes, les séquences d'acide nucléique (N1-n) étant liées à des particules (P).

14. Procédé selon l'une des revendications précédentes, les particules (P) présentant une grandeur de 30 nm à 3 mm.

15. Procédé selon l'une des revendications précédentes, les particules (P) étant des particules de silice, polystyrène, chlorure de polyvinyle, polyéthylène, nylon ou suspension de gel de silice.

16. Procédé selon l'une des revendications précédentes, la particule (P) étant une capside virale ou une particule ayant l'aspect de virus.

17. Procédé selon l'une des revendications précédentes, chacune des autres séquences d'acide nucléique (N'1-n) étant liée à un point défini d'une surface solide, de préférence sur une puce, une plaque Microtiter ou un film.

18. Procédé selon l'une des revendications précédentes, l'hybridation d'une section de séquence d'identification (IDS1-n) avec une section de séquence de détection complémentaire (IDP1-n) étant détectée au moyen de fluorescence.

19. Procédé selon l'une des revendications précédentes, au moins deux séquences d'acide nucléique (N1-n) étant ajoutées comme marquage de la substance (S1-n).

20. Procédé selon l'une des revendications précédentes, les séquences d'acide nucléique (N1-n) et/ou les autres séquences d'acide nucléique (N'1-n) étant fabriquées synthétiquement.

21. Procédé selon l'une des revendications précédentes, des chimères d'acides nucléiques et d'analogues d'acide nucléique, tels que PTO ou PNA, étant utilisées à la place de séquences d'acide nucléique ou d'autres séquences d'acide nucléique.
